(19) Europäisches Patentamt
European Patent Office
Office européen des brevets

(11) **EP 4 053 274 B1**

(12) **EUROPEAN PATENT SPECIFICATION**

(45) Date of publication and mention
of the grant of the patent:
**15.04.2026   Bulletin 2026/16**

(21) Application number: **20881744.5**

(22) Date of filing: **28.10.2020**

(51) International Patent Classification (IPC):
**C12N 9/90** *(2006.01)*        **C12N 9/16** *(2006.01)*
**C12N 9/12** *(2006.01)*        **C12P 19/02** *(2006.01)*
**C12P 19/24** *(2006.01)*

(52) Cooperative Patent Classification (CPC):
**C12N 9/90; C12P 19/02; C12P 19/24**

(86) International application number:
**PCT/KR2020/014808**

(87) International publication number:
**WO 2021/086010 (06.05.2021 Gazette 2021/18)**

(54) **FRUCTOSE-6-PHOSPHATE 3-EPIMERASE AND USE THEREOF**

FRUCTOSE-6-PHOSPHAT-3-EPIMERASE UND DEREN VERWENDUNG

FRUCTOSE-6-PHOSPHATE 3-ÉPIMÉRASE ET SON UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.10.2019   KR 20190134730**

(43) Date of publication of application:
**07.09.2022   Bulletin 2022/36**

(73) Proprietor: **Samyang Corporation**
**Jongno-gu**
**Seoul 03129 (KR)**

(72) Inventors:
• **HEO, Jinsol**
**Seongnam-si, Gyeonggi-do 13505 (KR)**
• **JOUNG, In-Suk**
**Yongin-si, Gyeonggi-do 16810 (KR)**
• **CHOI, Eunsoo**
**Seongnam-si, Gyeonggi-do 13588 (KR)**

(74) Representative: **Schön, Christoph**
**Dr. Schön, Neymeyr & Partner mbB**
**Bavariaring 26**
**80336 München (DE)**

(56) References cited:
WO-A1-2018/112139        KR-A- 20180 004 023
KR-A- 20190 079 351        KR-A- 20190 079 352
KR-B1- 101 318 422

• DATABASE PROTEIN [online] 2 June 2019
(2019-06-02), ANONYMOUS: "ribulose-
phosphate 3-epimerase [Clostridium lundense]",
XP055808256, retrieved from NCBI Database
accession no. WP_027625682
• DATABASE RefSeq [online] 19 June 2019
(2019-06-19), ANONYMOUS: "Ribulose-
phosphate 3-epimerase [Clostridium
tetanomorphum]", XP093092549, retrieved from
EBI Database accession no. WP_035150565
• DATABASE PROTEIN 2 June 2019 (2019-06-02),
ANONYMOUS: "ribulose-phosphate 3-epimerase
[Clostridium lundense]", XP055808256, retrieved
from NCBI Database accession no.
WP_027625682

Remarks:
The complete document including Reference
Table(s) and the Sequence Listing(s) can be
downloaded from the EPO website

EP 4 053 274 B1

**Description**

**[TECHNICAL FIELD]**

The present disclosure relates to a composition for producing allulose and a method for producing allulose.

**[BACKGROUND ART]**

Phosphorylated saccharide epimerase is an epimerase bound to the carbon of various phosphorylated saccharides, and ketohexose-6-phosphate epimerase can epimerize C3 or C4. The ketohexose may be one or more ketohexoses selected from the group consisting of fructose, allulose, sorbose, and tagatose.

Fructose-6-phosphate epimerases include 3-epimerase and 4-epimerase. Specifically, D-allulose-3-epimerase (EC 5.1.3.30) produces allulose-6-phosphate through 3-epimerization (epimerization at the C-3 position ) of fructose (D-fructose).

When producing allulose from fructose using the above enzyme, a certain level of reaction equilibrium exists between fructose used as a substrate and allulose as a product, so that the final conversion rate is only 20 to 35%. Therefore, when attempting to prepare high-purity allulose through a single reaction using the enzyme, an additional process of separating and removing fructose from the final reaction solution is required.

When industrially attempting to produce allulose from fructose as a raw material, the enzyme used must have high industrial production conditions, especially thermal stability, and the highest possible conversion rate. Further, saccharides are used as a substrate and browning of saccharides occurs easily under alkaline conditions, it is necessary to satisfy the conversion reaction condition to prevent browning of sugar as much as possible.

WP_027625682 discloses the amino acid sequence of ribulose-phosphate 3-epimerase [*Clostridium lundense*] having the accession number WP _027625682.

WP_035150565 discloses the amino acid sequence of ribulose-phosphate 3-epimerase [*Clostridium tetanomorphum*] having the accession number WP_035150565.

WO 2018/112139 A1 discloses a process for preparing allulose comprising converting fructose 6-phosphate to allulose 6-phosphate, catalyzed by allulose 6-phosphate 3-epimerase, and converting the allulose 6-phosphate to allulose, catalyzed by allulose 6-phosphate phosphatase.

Therefore, there is an urgent need for an enzyme that satisfies at least one of substrate conversion rate, thermal stability of the enzyme, and enzyme reaction conditions being suitable for industrial use, and that produces phosphorylated fructose using fructose as a raw material, and a method for producing phosphorylated fructose using the same.

**[DETAILED DESCRIPTION OF THE INVENTION]**

**[Technical Problem]**

The present invention provides a composition for producing allulose as defined in appended claim 1 and a method for producing allulose as defined in appended claim 8. Preferred embodiments are defined in the appended dependent claims.

**[Technical Solution]**

In order to achieve the above objects, the present invention provides a composition for producing allulose as defined in appended claim 1. The fructose-6-phosphate 3-epimerase protein has an amino acid sequence that has 70% or more, 80% or more, 90% or more, 95% or more, 97% or more, or 99% or more sequence homology to the amino acid sequence of SEQ ID NO: 1.

The fructose-6-phosphate 3-epimerase may be encoded by the nucleotide sequence of SEQ ID NO: 2, or a nucleotide sequence having at least 80%, 90%, 95%, 97%, or 99% or more homology to the nucleotide sequence of SEQ ID NO: 2.

As used herein, the term "homology" or "identity" refers to a degree of matching with a given amino acid sequence or nucleotide sequence, and it may be expressed as a percentage. In the present disclosure, a homology sequence having activity which is identical or similar to the given amino acid sequence or nucleotide sequence is expressed as "% homology".

The fructose-6-phosphate 3-epimerase catalyzes the 3-epimerization reaction of fructose 6-phosphate, and specifically, may perform 3-epimerization of fructose-6-phosphate converted to allulose-6-phosphate.

The fructose-6-phosphate 3-epimerase protein may be an enzyme derived from *Clostridium lundense,* and specifically, an enzyme derived from *Clostridium lundense* DSM 17049.

The reaction temperature range of the *Clostridium lundense*-derived fructose-6-phosphate 3-epimerase may be 40 to 70 °C, 45 to 75 °C, 45 to 77 °C, 50 to 70 °C, or 50 to 75 °C. The optimum temperature may be, for example, the result of the reaction proceeding for 5 minutes under the condition of pH 7.0. The optimum temperature condition for fructose-6-phosphate 3-epimerase is 60 °C, and it has an activity of 50% or more of the maximum enzyme activity in a wide temperature range of 40 to 70 °C.

The reaction pH range of the *Clostridium lundense*-derived fructose-6-phosphate 3-epimerase may be pH 6 to 8, pH 6 to 7.5, pH 6.5 to 8, pH 6.5 to 7.5, pH 7 to 8, or pH 7 to 7.5, and it has the maximum activity under the condition of pH 7.0 to 7.5, and has an activity of 80% or more of the maximum enzyme activity in the range of pH 6.0 to 8.0.

The maximum allulose production amount of the fructose-6-phosphate 3-epimerase derived from *Clostridium lundense* is 16 wt% or more, 18 wt% or more, 20 wt% or more, 25 wt% or more, 27 wt% or more, 30 wt% or more or 32% by weight or more. Specifically, the maximum allulose production amount of the enzyme may be measured for a reaction performed by adding 0.1 mg/ml of the enzyme to a dissolved solution of 20 g/L fructose-6-phosphate, and more specifically, may be measured by adding 0.1 mg/ml of the enzyme to a dissolved solution of 20 g/L fructose-6-phosphate an enzymatic reaction at pH 7.0 and 50 °C.

The maximum conversion rate of allulose may be calculated by Equation 1 below. The time of the enzymatic reaction to obtain the maximum conversion rate of allulose may be 8 hours or more, 10 hours or more, 12 hours or more, 14 hours or more, or 16 hours or more, and the upper limit of the reaction time may be 18 hours or less or 20 hours or less. The specific reaction time may be a range combining the lower limit and the upper limit, for example, may be 16 hours to 20 hours.

Allulose maximum conversion rate (%) = (allulose production amount (g/L) / fructose-6-phosphate input amount (g/L) * (allulose molecular weight / fructose-6-phosphate molecular weight) * 100    [Equation 1]

The ratio (% by weight) of allulose production of the saccharides in the enzymatic reaction product can be calculated by the following equation. The enzyme reaction time for obtaining the allulose production rate may be 2 hours to 6 hours, 3 hours to 6 hours, or 4 hours to 6 hours, for example, 4 hours to 6 hours.

Allulose production ratio (% by weight) = allulose production amount / (fructose production amount + allulose production amount) * 100    [Equation 2]

The fructose-6-phosphate 3-epimerase protein may be increased or decreased in its enzyme activity by metal ions. Specifically, the enzyme activity of the fructose-6-phosphate 3-epimerase protein is increased by Mn, Co and Ni ions. For example, it exhibits an activity as 1.1 times or more, 1.2 times or more, 1.3 times or more, or 1.5 times or more as compared with the condition without metal ions. In particular, Mn and Co ions exhibit an activity of 2 times or more, or 3 times or more, specifically 2 to 5 times, 2 to 4 times, 3 to 5 times, or 3 to 5 times as compared with the condition without metal ions. Fructose-6-phosphate 3-epimerase protein has a property that its activity is reduced by Ca, Cu, Fe, or Zn ions.

The fructose-6-phosphate 3-epimerase has 70% or more, 80% or more, 90% or more, 95% or more, 97% or more, or 99% or more sequence homology to the amino acid sequence of SEQ ID NO: 1.

For the *Clostridium lundense*-derived fructose-6-phosphate 3-epimerase protein, the amino acid sequence identity to *Ruminococcus sp.* AF14-10-derived ribulose-phosphate 3-epimerase (RuFP3E: amino acid sequence of SEQ ID NO: 5), *Clostridium sp.* DL-VIII-derived ribulose-phosphate 3-epimerase (CDFP3E: amino acid sequence of SEQ ID NO: 7), and *Paenibacillus kribbensis*-derived ribulose-phosphate 3-epimerase (PkFP3E: amino acid sequence of SEQ ID NO: 9) was analyzed, and as a result, the sequence identity to RuFP3E was 59.05%, the sequence identity to CDFP3E was 63%, the amino acid sequence identity to PkFP3E was 60.96%, and the sequence homology to the amino acid sequence of SEQ ID NO: 1 was less than 70%.

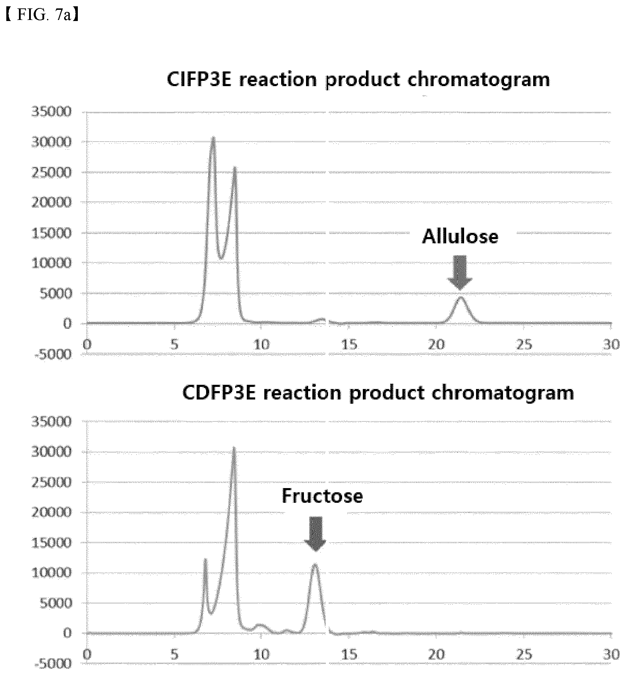

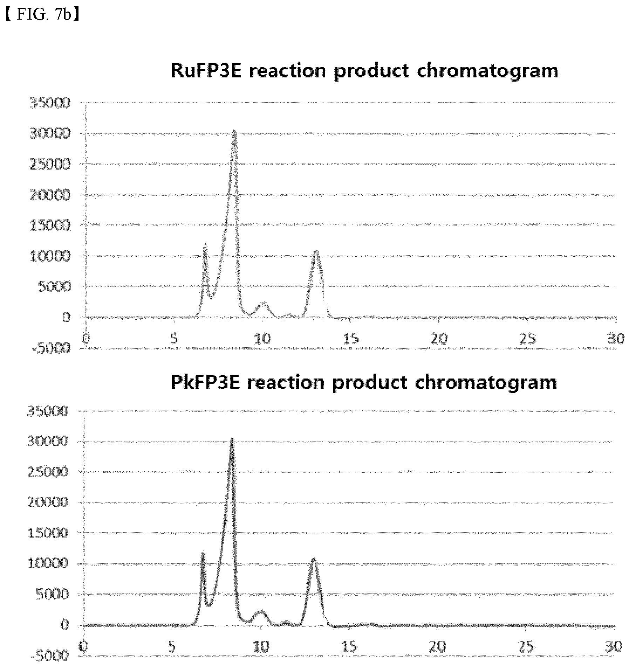

Further, as a result of HPLC analysis of the conversion activity of fructose 6-phosphate to allulose 6-phosphate, an allulose peak was indentified in the ClFP3E enzyme having SEQ ID NO: 1, but an allulose peak was not identified in the RuFP3E, CDFP3E and PkFP3E. Thus, because other candidate enzymes other than the ClFP3E enzyme had no activity against F6P, it can be confirmed that when phosphatase is treated, only fructose in the form in which phosphate group is removed from F6P, which is the initial substrate of the reaction step, is generated (Fig. 7a, 7b).

As used herein, the term "transformation" refers to a process of introducing into a host cell a vector including a nucleic acid encoding a target protein, thereby enabling the expression of the protein encoded by the nucleic acid in the host cell. For the transformed nucleic acid, it does not matter whether the transformed nucleic acid is inserted into the chromosome of a host cell and located therein or located outside the chromosome, as long as it can be expressed in the host cell, and both cases are included. Additionally, the nucleic acid includes DNA and RNA which encode the target protein. The nucleic acid may be inserted in any form as long as it can be introduced into a host cell and expressed therein. For example, the nucleic acid may be introduced into a host cell in the form of an expression cassette, which is a gene construct including all essential elements required for self-expression. The expression cassette may conventionally include a promoter operably linked to the nucleic acid, a transcription termination signal, a ribosome-binding domain, and a translation termination signal. The expression cassette may be in the form of an expression vector capable of self-replication. Additionally, the nucleic acid may be introduced into a host cell as it is and operably linked to a sequence essential for its expression in the host cell.

Additionally, as used herein, the term "operably linked" refers to a functional linkage between a promoter sequence, which initiates and mediates the transcription of the nucleic acid encoding the target protein of the present disclosure, and the above gene sequence.

The method for transforming the vector includes any method of introducing a nucleic acid into a cell, and may be carried out by selecting a suitable standard technique known in the art according to a host cell. Examples of the method may include electroporation, calcium phosphate ($CaPO_4$) precipitation, calcium chloride ($CaCl_2$) precipitation, micro-injection, a polyethyleneglycol (PEG) technique, a DEAE-dextran technique, a cationic liposome technique, a lithium acetate-DMSO technique, etc.

As the host cell, it is preferable to use a host having a high efficiency of introducing DNA and a high efficiency of expressing the introduced DNA. For example, it may be E. coli.

The composition for producing allulose as defined in appended claim 1 comprises at least one selected from the group consisting of the fructose-6-phosphate 3-epimerase as defined in appended claim 1, a microorganism expressing the enzyme protein, a transgenic microorganism expressing the enzyme protein, a microbial cell of the microorganism, a cell lysate of the microbial cell of the microorganism, a culture of the microorganism, a culture supernatant of the microorganism, a concentrate of the culture supernatant of the microorganisms and their powders.

The culture contains an enzyme produced from a microorganism that produces fructose-6-phosphate 3-epimerase, and may be in a cell-free form with or without the strain. The cell lysate means a lysate obtained by disrupting the microbial cells of a microorganism producing fructose-6-phosphate 3-epimerase or a supernatant obtained by centrifuging the lysate, and contains an enzyme produced from a microorganism that produces the polyphosphate-dependent glucose kinase.

The culture of the strain contains the enzyme produced from the microorganism producing the fructose-6-phosphate 3-epimerase, and may be in a cell-free form with or without the microbial cells. Unless otherwise stated herein, the microorganisms used to produce fructose-6-phosphate 3-epimerase means inclusioin of at least one selected from the group consisting of the microbial cell of the strain, the culture of the strain, the lysate of the microbial cell, the supernatant of the lysate, and extracts thereof.

The method for producing allulose, according to the present disclosure is environmentally friendly, because it uses an enzyme obtained from a microorganism. The conversion of allulose from fructose by using a simple enzymatic reaction of an new method, significantly reduces production costs and maximizes the production effect.

The composition for producing allulose according to the present disclosure further includes one or more metal ions selected from the group consisting of Mn, Co, and Ni ions. The concentration of the metal ion may be 0.5 mM to 20 mM, and for example, it may be 0.5 mM to 10 mM, 1.0 mM to 10 mM, 1.5 mM to 8.0 mM, 2.0 mM to 8.0 mM, 3.0 mM to 7.0 mM, 4.0 mM to 6.0 mM, or 0.2 mM to 10 mM.

When producing allulose using the composition for producing allulose, the reaction temperature and reaction pH conditions using the enzyme or the enzyme-producing microorganism are the same as described above in the reaction temperature and reaction pH conditions of the enzyme.

The composition for producing allulose includes at least one selected from the group consisting of an epimerase protein of fructose-6-phosphate, a transgenic microorganism expressing the enzyme protein, a microbial cell of the microorganism, a microbial cell lysate of the microorganism, a culture of the microorganism, a culture supernatant of the microorganism, a concentrate of the culture supernatant of the microorganisms and their powder.

The fructose-6-phosphate is preferably obtained by hexokinase treatment of fructose or a fructose-containing material.

The fructose-6-phosphate may be prepared from glucose-6-phosphate, and the composition for producing allulose may further include a glucose-6-phosphate isomerase converting glucose-6-phosphate to fructose-6-phosphate by isomerizing glucose-6-phosphate.

The glucose-6-phosphate can be prepared by direct phosphorylation of glucose, or may be converted from glucose-1-phosphate. Glucose may be obtained by treating starch or starch hydrolyzate, for example, dextrin with gluconeogenesis amylase, and glucose-1-phosphate can be obtained by treating the glucose with a phosphorylation enzyme. The composition for producing ketohexose may further include an enzyme system for producing glucose-6-phosphate.

The composition of the present disclosure for producing allulose may further include (a) (i) starch, maltodextrin, sucrose, or a combination thereof; (ii) phosphate; (iii) allulose -6-phosphate phosphatase; (iv) glucose-6-phosphate-isomerase; (v) phosphoglucomutase or glucose phosphorylase; and (vi) $\alpha$-glucanophosphorylase, starch phosphorylase, maltodextrin phosphorylase, sucrose phosphorylase, $\alpha$-amylase, pullulanase, isoamylase, glucoamylase or sucrase; or

(b) a microorganism expressing any of the enzymes described in item (a) or a culture of the microorganism.

Specifically, the starch/maltodextrin phosphorylase (EC 2.4.1.1) and α-glucanophosphorylase may include any proteins as long as these are proteins that are subjected to phosphoryl transfer hosphate to glucose, thereby having the activity of producing glucose-1-phosphate from starch or maltodextrin.

The sucrose phosphorylase (EC 2.4.1.7) may include any protein as long as it is a protein that is subj ected to transfer phosphate to glucose, thereby having the activity of producing glucose-1-phosphate from sucrose.

The α-amylase (EC 3.2.1.1), pullulanase (EC 3.2.1.41), glucoamylase (EC 3.2.1.3), and isoamylase, which are enzymes for starch saccharification, may include any proteins as long as these are proteins having the activity of converting starch or maltodextrin to glucose.

The sucrase (EC 3.2.1.26) may include any protein as long as it is a protein having the activity of converting sucrose to glucose. The phosphoglucomutase (EC 5.4.2.2) may include any protein as long as it is a protein having the activity of converting glucose-1-phosphate to glucose-6-phosphate. The glucokinase may include any protein as long as it is a protein capable of transferring phosphate to glucose, thereby having the activity of converting to glucose-6-phosphate. Specifically, the glucokinase may be a polyphosphate-dependent glucokinase.

The glucose-6-phosphate isomerase may include any protein as long as it has an activity to convert glucose-6-phosphate to fructose-6-phosphate.

The allulose-6-phosphate dephosphorelyation enzyme may include any protein as long as it is a protein having the activity of converting allulose-6-phosphate to allulose. More specifically, the allulose-6-phosphate phosphatase may be a protein having an activity of irreversibly converting allulose-6-phosphate to allulose. The composition for producing allulose may further include phytase, which performs a dephosphorylation reaction in allulose-6-phosphate, for example, allulose-6-phosphate phosphatase. The composition for producing allulose may further include allulose-6-phosphate phosphatase, microorganisms expressing the allulose-6-phosphate phosphatase or a culture of a microorganism expressing the allulose-6-phosphate phosphatase.

The method may further include preparing fructose-6-phosphate from fructose or a fructose-containing material using hexokinase, and/or may further include contacting phosphatase with a microorganism expressing the same, or a culture of the microorganism to remove the phosphate.

The step of removing the phosphate can be performed using allulose-6-phosphate phosphatase, a microorganism expressing the allulose-6-phosphate phosphatase or a culture of a microorganism expressing the allulose-6-phosphate phosphatase to produce allulose. The allulose 6-phosphate can remove a phosphate group by other enzymes or chemical methods.

The present invention provides a method for producing allulose as defined in appended claim 8, which comprises using the composition for producing allulose of the present invention to convert fructose-6-phosphate to allulose-6-phosphate.

After the step of converting fructose-6-phosphate to allulose-6-phosphate, the preparation method may further include a step of contacting allulose-6-phosphate with allulose-6-phosphate phosphatase, a microorganism expressing the allulose-6-phosphate phosphatase, or a culture of a microorganism expressing the allulose-6-phosphate phosphatase, thereby converting the allulose-6-phosphate to allulose.

Before the step of converting fructose-6-phosphate to allulose-6-phosphate, the preparation method may further include a step of contacting glucose-6-phosphate with glucose-6-phosphate-isomerase, a microorganism expressing the glucose-6-phosphate-isomerase, or a culture of a microorganism expressing the glucose-6-phosphate-isomerase, thereby converting the glucose-6-phosphate to fructose-6-phosphate.

Further, the preparation method may further include, before the step of converting fructose-6-phosphate to fructose-6-phosphate, a step of contacting glucose-6-phosphate with phosphoglucomutase, a microorganism expressing the phosphoglucomutase or a culture of a microorganism expressing the phosphoglucomutase, thereby converting the glucose-1-phosphate to glucose-6-phosphate.

Further, the preparation method may further include, before the step of converting glucose-6-phosphate to fructose-6-phosphate, a step of contacting glucose with a glucose kinase, a microorganisms expressing the glucose kinase or a culture of a microorganism expressing the glucose kinase, and phosphate, thereby converting the glucose to glucose-6-phosphate.

The preparation method may further include, before the step of converting glucose-1-phosphate to glucose-6-phosphate, a step of contacting starch, maltodextrin, sucrose or a combination thereof with α-glucan phosphorylase, starch phosphorylase, maltodextrin phosphorylase or sucrose phosphorylase; a microorganism expressing the phosphorylase; or a culture of a microorganism expressing the phosphorylase, and phosphate, thereby converting the starch, maltodextrin, sucrose or a combination thereof to glucose-1-phosphate.

The preparation method may further include, before the step of converting the glucose to glucose-6-phosphate, a step of contacting starch, maltodextrin, sucrose or a combination thereof with α-amylase, pullulanase, glucoamylase, sucrase or isoamylase; a microorganism expressing the amylase, pullulanase or sucrase; or a culture of microorganisms expressing the amylase, pullulanase or sucrase, thereby converting the starch, maltodextrin, sucrose or a combination thereof to glucose.

The preparation method may further include a step of contacting glucose with 4-$\alpha$-glucanotransferase, a microorganism expressing the 4-$\alpha$-glucanotransferase or a culture of a microorganism expressing the 4-$\alpha$-glucano-transferase, thereby converting the glucose to starch, maltodextrin or sucrose.

The allulose produced according the preparation method can be usefully used by adding it to functional foods and pharmaceuticals.

[ADVANTAGEOUS EFFECTS]

The composition according to the present invention satisfies at least one of the properties of high enzyme conversion rate, acidic or neutral reaction pH conditions, and high thermal stability, and thus, can be usefully used for the production of allulose using fructose-6-phosphate 3-epimerase on an industrial scale.

**[BRIEF DESCRIPTION OF THE DRAWINGS]**

Fig. 1 is an electrophoresis photograph confirming the expression and purification of fructose-6-phosphate 3-epimerase protein;

Fig. 2 shows the results of BIO-LC analysis of fructose-6-phosphate 3-epimerase protein;

Fig. 3 shows the results of LC analysis after dephosphorylating the phosphorylated saccharide in the reaction solution through the enzymatic reaction of allulose-6-phosphate phosphatase;

Fig. 4 is a graph showing the results of analyzing the temperature characteristics of fructose-6-phosphate 3-epimerase protein;

Fig. 5 is a graph showing the results of analyzing the pH characteristics of fructose-6-phosphate 3-epimerase protein;

Fig. 6 is a graph showing the effect of metal ions of fructose-6-phosphate 3-epimerase protein; and

Fig. 7a and Fig. 7b is an HPLC analysis result of a reaction product obtained after performing allulose production using three types of conventionally known ribulose-phosphate 3-epimerase.

**[DETAILED DESCRIPTION OF THE EMBODIMENTS]**

The present disclosure will be described in more detail with reference to the following examples.

**Example 1: Production of fructose-6-phosphate 3-epimerase**

Candidate enzymes expected to function as fructose-6-phosphate 3-epimerase were screened, and as the enzyme expected to show the best effect, polynucleotide (SEQ ID NO: 2) encoding the amino acid sequence (SEQ ID NO: 1) of the enzyme (CIFP3E) derived from *Clostridium lundense* DSM 17049 strain was obtained by requesting gene synthesis through IDT gene synthesis. A primer was designed based on the synthesized CIFP3E DNA sequence of SEQ ID NO: 2, and PCR was performed to amplify the nucleotide sequence of the gene. The forward and reverse primer sequences used for PCR amplification are as follows.

[Table 1]

| Primer name | Sequence (5'->3') | SEQ ID NO: |
|---|---|---|
| pET21_CIRP3E_F | tatacatatgAAATACGGCTTCGCACC | 3 |
| pET21_CIRP3E_R | ggtgctcgagTTCTTTGTAGCTCAGGCTGTA | 4 |

The CIFP3E gene obtained in large quantities was introduced into the pET21a vector using restriction enzymes NdeI and XhoI to prepare pET21_CIFP3E, which was transformed into *Escherichia coli* ER2566 strain. Recombinant *E. coli* for enzyme protein expression was obtained as colonies on an agar plate prepared in LB medium containing 50 $\mu$g/ml ampicillin.

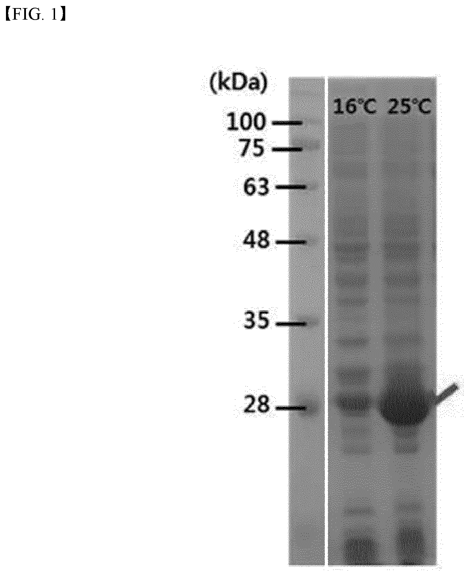

After seed culture in 4ml LB medium, the main culture was performed in 100ml LB medium. The culture conditions were incubated at 37°C and 200 rpm until the absorbance value at 600 nm was 0.6, and then 0.1 mM IPTG was added thereto to induce expression of the target protein. After induction, the strain was cultured at 25°C for about 16 hours, and then centrifuged to recover the cells. The recovered cells were suspended in a lysis buffer (50 mM sodium phosphate (pH 7.0) buffer, 300 mM NaCl, 10 mM imidazole), and the cells were disrupted using a beadbeater. The overexpression of the target protein ClFP3E from the cell disruption solution was confirmed by SDS-PAGE gel analysis. The results of overexpression analysis of the target protein ClFP3E are shown in Fig. 1. The molecular weight of ClFP3E confirmed by SDS-PAGE gel analysis was about 28 KDa.

Additionally, after removing the cell pellet, only the cell supernatant was obtained and bound to a Ni-NTA column (Ni-NTA superflow, Qiagen), and then proteins not bound to the column were removed with a washing buffer (50 mM sodium phosphate (pH 7.0) buffer, 300 mM NaCl, 20 mM imidazole). As a final step, the protein of interest was eluted with an elution buffer (50 mM sodium phosphate (pH 7.0) buffer, 300 mM NaCl, 200 mM imidazole). The finally secured protein was converted to 50mM sodium phosphate buffer (pH 7.0) and stored for subsequent use.

**Example 2**: **Evaluation of the conversion activity of enzymes**

0.1mg/ml of the purified ClFP3E enzyme obtained in Example 1 was added to a solution in which 20g/L of fructose-6-phosphate was dissolved in 50mM sodium phosphate (pH 7.0) buffer, and the enzymatic reaction was performed at 50 °C.

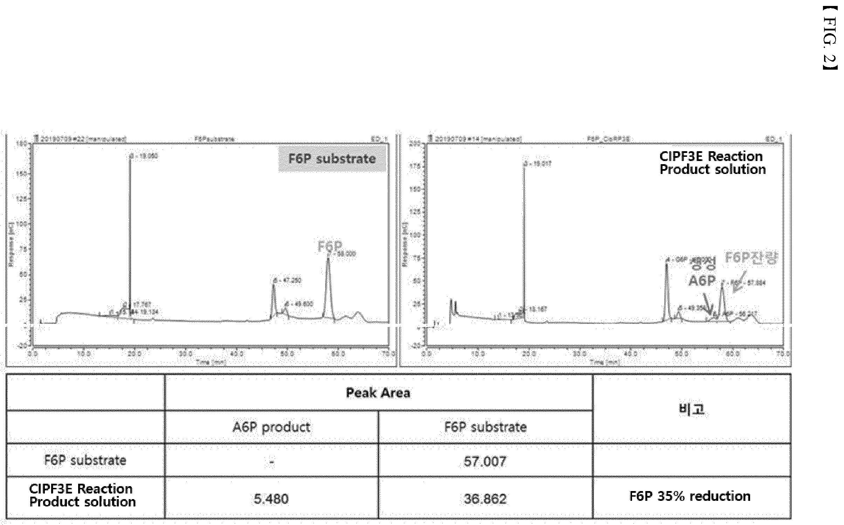

The analysis of the enzymatic reaction solution confirmed a newly produced substance as compared with the substrate through Bio-LC analysis. However, since the allulose-6-phosphate standard did not exist, accurate confirmation was not possible, and thus, after further treatment with a phosphatase enzyme of allulose-6-phosphate (A6PP), the resulting allulose was finally confirmed. Bio-LC analysis confirmed that during the ClFP3E enzyme reaction, a decrease in F6P and a new peak thought to be A6P were generated. The results of the Bio-LC analysis are shown in Fig. 2 below.

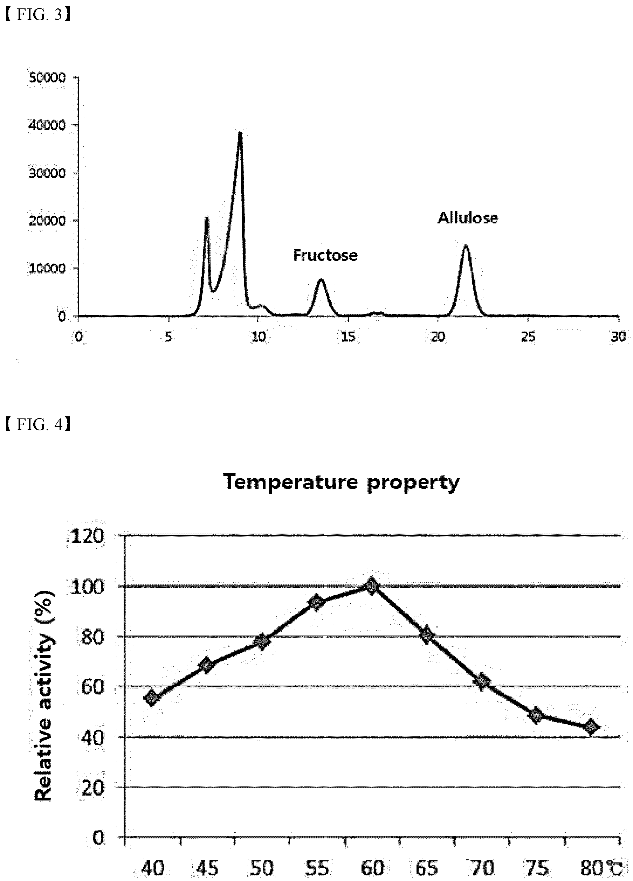

The result of LC analysis after dephosphorylating the phosphorylated saccharide in the reaction solution through the A6PP enzymatic reaction is as follows. Analysis was performed at a temperature of 80°C and a flow rate of 0.6ml/min using Aminex HPX-87C column, and the results are shown in Fig. 3. As a result of the analysis, fructose and allulose could be confirmed.

The final conversion rate of ClFP3E calculated by quantifying the amount of allulose produced, which is the final product of the reaction solution dephosphorylated by the Arlos -6-phosphate phosphatase (A6PP) enzymatic reaction, was calculated to be 34.3%. T The reaction product of this example is the final conversion of ClFP3E obtained by performing the enzymatic reaction for 16 hours, and the maximum conversion rate of allulose is calculated according to the following equation.

Allulose maximum conversion rate (%) = (allulose production amount (g/L) / fructose-6-phosphate input amount (g/L) * (allulose molecular weight / fructose-6-phosphate molecular weight) * 100    [Equation 1]

**Example** 3: **Analysis of temperature characteristics of enzymes**

To determine the effect of temperature on ClFP3E enzyme activity, 10 g/L of fructose-6-phosphate was dissolved in 50 mM sodium phosphate (pH 7.0) buffer, and then 0.01 mg/ml of ClFP3E purified protein was added thereto, and the reaction was performed at various temperature conditions between 40 and 80 °C for 5 minutes.

Then, the A6PP enzyme was added to dephosphorylate all reaction compositions, and then, the amount of allulose produced was quantitatively analyzed through HPLC analysis. The relative activity of the enzyme according to the reaction temperature is shown in FIG. 4 based on the activity at 60°C where the highest activity was measured.

As a result of the experiment, it was confirmed that the optimum temperature condition for ClFP3E was confirmed to be 60°C, and the activity was 50% or more of the maximum enzyme activity in a wide temperature condition range of 40 to 70°C.

**Example 4: Analysis of pH characteristics of enzymes**

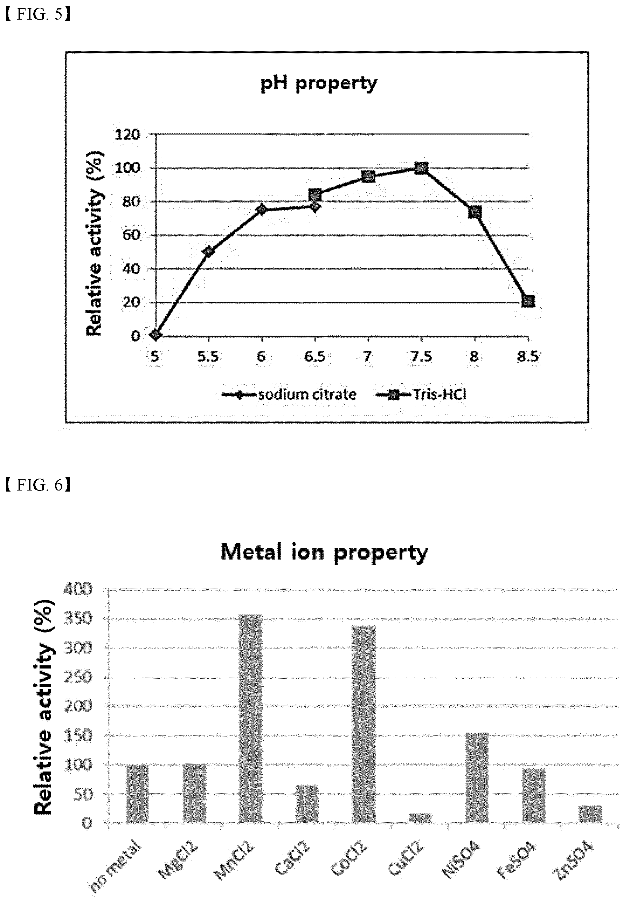

To confirm the effect of pH on ClFP3E enzyme activity, 10 g/L of fructose-6-phosphate was dissolved in a buffer of pH 5.0~8.5 (pH 5.0-6.5, sodium citrate / pH 6.5~8.5, Tris-HCl), 0.01 mg/ml of purified ClFP3E protein was added thereto, and the enzymatic reaction was performed at a temperature of 60 °C for 5 minutes. Then, A6PP enzyme was added to dephosphorylate all reaction compositions, and then allulose production amount was quantitatively analyzed through HPLC analysis. The result of the enzyme activity according to the reaction pH is shown in Fig. 5 as a relative activity based on pH 7.5, which had the best activity.

As a result of the experiment, it was possible to confirm the maximum activity at pH 7.0 to 7.5, and it was confirmed that it had 80% or more of the maximum enzyme activity in the range of pH 6.0 to 8.0.

**Example 5: Analysis of the effect of metal ions on enzymes**

In order to confirm the activity of ClFP3E according to the type of metal ion added during the reaction, 10 g/L of fructose-6-phosphate was dissolved in 50 mM sodium phosphate (pH 7.0) buffer, 5 mM of each metal ion ($MgCl_2$, $MnCl_2$, $CaCl_2$, $CoCl_2$, $CuCl_2$, $NiSO_4$, $FeSO_4$, $ZeSO_4$) was added. 0.01 mg/ml of ClFP3E purified protein was added to the reaction buffer containing each metal ion, and the reaction was carried out at a temperature of 60°C for 5 minutes.

Then, the A6PP enzyme was added to dephosphorylate all reaction compositions, and then, the amount of allulose produced was quantitatively analyzed through HPLC analysis. The relative activity of the enzyme depending on the type of the metal ion is shown in Fig. 6 based on the experimental group in which no metal ion was added.

As a result of the experiment, when $MnCl_2$ and $CoCl_2$ were added, it was confirmed that the activity was three times higher than the condition in which no metal ions were added. It was found that the ClFP3E enzyme is an enzyme using manganese and cobalt as cofactors. Also, the activity was increased even by nickel. It was $CaCl_2$, $CuCl_2$, $FeSO_4$, $ZeSO_4$ that decreased the enzyme activity.

**Comparative Example 1: Analysis of allulose production using ribulose-phosphate 3-epimerase**

Polynucleotides of *Ruminococcus sp.* AF14-10-derived ribulose-phosphate 3-epimerase (RuFP3E: amino acid sequence of SEQ ID NO: 5 and nucleic acid sequence of SEQ ID NO: 6), *Clostridium sp.* DL-VIII-derived ribulose-phosphate 3-epimerase (CDFP3E: amino acid sequence of SEQ ID NO: 7 and nucleic acid sequence of SEQ ID NO: 8), *Paenibacillus kribbensis*-derived ribulose-phosphate 3-epimerase (PkFP3E: amino acid sequence of SEQ ID NO: 9 and nucleic acid sequence of SEQ ID NO: 10) were obtained by requesting gene synthesis.

As a result of analyzing the amino acid sequence homology to the amino acid sequence (SEQ ID NO: 1) of fructose-6-phosphate 3-epimerase (ClFP3E) derived from the *Clostridium lundense* DSM 17049 strain, the amino acid sequence identity to RuFP3E having the amino acid sequence of SEQ ID NO: 5 was 59.05%, the amino acid sequence identity to CDFP3E having the amino acid sequence of SEQ ID NO: 7 was 63%, and the amino acid sequence identity to PkFP3E having the amino acid sequence of SEQ ID NO: 9 was 60.96%.

Based on the synthesized ClFP3E DNA sequence of SEQ ID NO: 2, a large amount of genes were obtained in substantially the same method as in Example 1. In substantially the same method as in Example 1, the obtained gene was introduced into and expressed in *E. coli* and then the protein of interest was eluted. Finally, the obtained protein was converted to 50mM sodium phosphate buffer (pH 7.0) and stored for subsequent use.

In order to analyze the conversion activity of fructose 6-phosphate to allulose 6-phosphate, an enzymatic reaction using fructose-6-phosphate as a substrate was performed using the obtained enzyme in the same method as in Example 2. After the enzymatic reaction, a dephosphorylation reaction using an allulose-6-phosphate phosphatase (A6PP) enzyme was performed, and then, the amount of produced allulose was measured for the reaction product solution using High-Performance Liquid Chromatography (HPLC).

As for HPLC analysis conditions, RID (Refractive Index Detector Agilent 1260 RID) of HPLC (Agilent, USA) equipped with an Aminex HPX-87C column (BIO-RAD) was used. Water was used as the mobile phase solvent, and the temperature was 80°C and the flow rate was 0.6 ml/min. The results of the HPLC analysis are shown in Fig. 7a and 7b. The reaction product of this example was obtained by performing the enzymatic reaction for 4 hours to obtain the allulose conversion rate of the enzyme. As an experiment for screening the FP3E candidate enzyme, the reaction solution in which the reaction was stopped at the intermediate stage of allulose production was analyzed. In Fig. 7a and Fig. 7b, the allulose conversion rate of ClFP3E was 16%, and the reaction proceeded to about 50% level of the maximum conversion rate of Example 2. In this Example, the allulose production ratio of total reaction products was 75%. The alullose production rate is calculated by the following Equation.

Allulose production ratio (% by weight) = allulose production amount / (fructose production amount + allulose production amount) * 100

As shown in Fig. 7a and Fig. 7b, no allulose peak was confirmed in other candidate enzymes except for the ClFP3E enzyme of Example 1. Therefore, because other candidate enzymes other than the ClFP3E enzyme had no activity against F6P, it was confirmed that when phosphatase was treated, only fructose of a dephosphorylated from produced from F6P as an initial substrate of the reaction step, was generated.

**Claims**

1. A composition for producing allulose, comprising at least one selected from the group consisting of an epimerase protein of fructose-6-phosphate which has at least 70% of amino acid sequence identity to the amino acid sequence of SEQ ID NO: 1 and converts fructose-6-phosphate to allulose-6-phosphate, a microorganism expressing the epimerase protein, a transgenic microorganism expressing the epimerase protein, a microbial cell of the microorganism, cell lysate of the microbial cell of the microorganism, a culture of the microorganism, a culture supernatant of the microorganism, a concentrate of the culture supernatant of the microorganisms and their powders, wherein the composition further comprises one or more metal ions selected from the group consisting of manganese ion, cobalt ion and nickel ion.

2. The composition according to claim 1, wherein the epimerase protein is encoded by a nucleotide sequence having at least 80% of nucleotide sequence identity to the nucleotide sequence of SEQ ID NO: 2.

3. The composition according to claim 1, wherein the epimerase protein is derived from *Clostridium lundense,* and has an enzyme reaction temperature of 40 to 70°C and an enzyme reaction pH of pH 6 to 8.

4. The composition according to claim 1, wherein the epimerase protein activity is increased by a manganese ion, a cobalt ion, or a nickel ion.

5. The composition for producing allulose according to any one of claims 1 to 4, wherein the composition further comprises a phosphatase enzyme of allulose 6-phosphate, a microorganism expressing the same, or a culture of the microorganism.

6. The composition for producing allulose according to any one of claims 1 to 4, wherein the composition further comprises an isomerase converting glucose-6-phosphate to fructose-6-phosphate, a microorganism expressing the same, or a culture of the microorganism.

7. The composition for producing allulose according to any one of claims 1 to 4, wherein the composition further comprises:

   (a) (i) starch, maltodextrin, sucrose, or a combination thereof; (ii) phosphate; (iii) allulose-6-phosphate phosphase; (iv) glucose-6-phosphate isomerase; (v) phosphoglucomutase or glucose phosphorylase; and (vi) $\alpha$-glucanophosphorylase, starch phosphorylase, maltodextrin phosphorylase, sucrose phosphorylase, $\alpha$-amylase, pullulanase, isoamylase, glucoamylase or sucrase; or
   (b) a microorganism expressing the enzyme of item (a) or a culture of the microorganism, and

   wherein the composition further comprises a phosphatase enzyme of allulose 6-phosphate.

8. A method for producing allulose, comprising a step of converting fructose-6-phosphate to allulose-6-phosphate using the composition for producing allulose as set forth in any one of claims 1 to 4.

9. The method according to claim 8, which further comprises a step of converting allulose-6-phosphate to allulose by contacting the allulose-6-phosphate with phytase, a microorganism expressing the same, or a culture of the microorganism.

10. The method according to claim 8, which the step of converting fructose-6-phosphate to allulose-6-phosphate is performed at a reaction temperature of 40 to 70°C and a reaction pH of pH 6 to 8.

11. The method according to claim 8, which further comprises a step of preparing fructose-6-phosphate from fructose or a fructose-containing material using hexokinase.

**Patentansprüche**

1. Zusammensetzung zur Herstellung von Allulose, wobei die Zusammensetzung mindestens eine Komponente, die aus der Gruppe ausgewählt ist, die aus einem Epimerase-Protein von Fructose-6-phosphat, das mindestens 70% Aminosäuresequenz-Identität mit der Aminosäuresequenz von SEQ ID Nr.: 1 aufweist und Fructose-6-phosphat in Allulose-6-phosphat umwandelt, einem Mikroorganismus, der das Epimerase-Protein exprimiert, einem transgenen Mikroorganismus, der das Epimerase-Protein exprimiert, einer mikrobiellen Zelle des Mikroorganismus, einem Zell-Lysat der mikrobiellen Zelle des Mikroorganismus, einer Kultur des Mikroorganismus, einem Kulturüberstand des Mikroorganismus, einem Konzentrat des Kulturüberstands der Mikroorganismen und deren Pulvern besteht, umfasst, wobei
die Zusammensetzung des Weiteren ein oder mehrere Metallionen, die aus der Gruppe ausgewählt sind, die aus Manganionen, Kobaltionen und Nickelionen besteht, umfasst.

2. Zusammensetzung gemäß Anspruch 1, wobei das Epimerase-Protein durch eine Nukleotidsequenz codiert ist, die mindestens 70% Nukleotidsequenz-Identität mit der Nukleotidquenz von SEQ ID Nr.: 2 aufweist

3. Zusammensetzung gemäß Anspruch 1, wobei das Epimerase-Protein von *Clostridium lundense* herrührt und eine Temperatur der enzymatischen Reaktion von 40 bis 70 °C und einen pH-Wert der enzymatischen Reaktion von pH 6 bis 8 aufweist.

4. Zusammensetzung gemäß Anspruch 1, wobei die Aktivität des Epimerase-Proteins durch ein Manganion, ein Kobaltion oder ein Nickelion erhöht ist.

5. Zusammensetzung zur Herstellung von Allulose gemäß einem der Ansprüche 1 bis 4, wobei die Zusammensetzung des Weiteren ein Phosphatase-Enzym von Allulose-6-phosphat, einen Mikroorganismus, der selbiges exprimiert, oder eine Kultur des Mikroorganismus umfasst.

6. Zusammensetzung zur Herstellung von Allulose gemäß einem der Ansprüche 1 bis 4, wobei die Zusammensetzung des Weiteren eine Isomerase, die Glucose-6-phosphat in Fructose-6-phosphat umwandelt, einen Mikroorganismus, der selbige exprimiert, oder eine Kultur des Mikroorganismus umfasst.

7. Zusammensetzung zur Herstellung von Allulose gemäß einem der Ansprüche 1 bis 4, wobei die Zusammensetzung des Weiteren Folgendes umfasst:

   (a) (i) Stärke, Maltodextrin, Saccharose oder eine Kombination hiervon, (ii) Phosphat, (iii) Allulose-6-phosphat-Phosphatase, (iv) Glucose-6-phosphat-Isomerase, (v) Phosphoglucomutase oder Glucosephosphorylase, und (vi) α-Glucanophosphorylase, Stärkephosphorylase, Maltodextrinphosphorylase, Saccharosephosphorylase, α-Amylase, Pullulanase, Isoamylase, Glucoamylase oder Sucrase, oder
   b) einen Mikroorganismus, der das Enzym aus Punkt (a) exprimiert, oder eine Kultur des Mikroorganismus, und

   wobei die Zusammensetzung des Weiteren ein Phosphatase-Enzym von Allulose-6-phosphat umfasst.

8. Verfahren zur Herstellung von Allulose, das einen Schritt des Umwandelns von Fructose-6-phosphat in Allulose-6-phosphat unter Verwendung der Zusammensetzung zur Herstellung von Allulose gemäß Darlegung in einem der Ansprüche 1 bis 4 umfasst.

9. Verfahren gemäß Anspruch 8, das des Weiteren einen Schritt des Umwandelns von Allulose-6-phosphat in Allulose durch In-Kontakt-Bringen des Allulose-6-phosphats mit Phytase, einem Mikroorganismus, der selbige exprimiert, oder einer Kultur des Mikroorganismus umfasst.

10. Verfahren gemäß Anspruch 8, bei dem der Schritt des Umwandelns von Fructose-6-phosphat in Allulose-6-phosphat bei einer Reaktionstemperatur von 40 bis 70 °C und einem pH-Wert der Reaktion von pH 6 bis pH 8 durchgeführt wird.

11. Verfahren gemäß Anspruch 8, das des Weiteren einen Schritt des Herstellens von Fructose-6-phosphat aus Fructose oder einem fructosehaltigen Material unter Verwendung von Hexokinase umfasst.

**Revendications**

1. Composition pour la production d'allulose, comprenant au moins un élément choisi dans le groupe constitué par une protéine épimérase de fructose-6-phosphate qui a une identité de séquence d'acides aminés d'au moins 70 % avec la séquence d'acides aminés de SEQ ID NO : 1 et convertit le fructose-6-phosphate en allulose-6-phosphate, un micro-organisme exprimant la protéine épimérase, un micro-organisme transgénique exprimant la protéine épimérase, une cellule microbienne du micro-organisme, un lysat cellulaire de la cellule microbienne du micro-organisme, une culture du micro-organisme, un surnageant de culture du micro-organisme, un concentré du surnageant de culture des micro-organismes et leurs poudres, dans laquelle
la composition comprend en outre un ou plusieurs ions métalliques choisis dans le groupe constitué par l'ion manganèse, l'ion cobalt et l'ion nickel.

**2.** Composition selon la revendication 1, dans laquelle la protéine épimérase est codée par une séquence nucléotidique ayant une identité de séquence nucléotidique d'au moins 80 % avec la séquence nucléotidique de SEQ ID NO : 2.

**3.** Composition selon la revendication 1, dans laquelle la protéine épimérase est dérivée de *Clostridium lundense,* et a une température de réaction enzymatique de 40 à 70 °C et un pH de réaction enzymatique de pH 6 à 8.

**4.** Composition selon la revendication 1, dans laquelle l'activité de la protéine épimérase est augmentée par un ion manganèse, un ion cobalt ou un ion nickel.

**5.** Composition pour la production d'allulose selon l'une quelconque des revendications 1 à 4, dans laquelle la composition comprend en outre une enzyme phosphatase d'allulose-6-phosphate, un micro-organisme exprimant celle-ci ou une culture du micro-organisme.

**6.** Composition pour la production d'allulose selon l'une quelconque des revendications 1 à 4, dans laquelle la composition comprend en outre une isomérase convertissant le glucose-6-phosphate en fructose-6-phosphate, un micro-organisme exprimant celle-ci ou une culture du micro-organisme.

**7.** Composition pour la production d'allulose selon l'une quelconque des revendications 1 à 4, dans laquelle la composition comprend en outre :

(a) (i) amidon, maltodextrine, saccharose ou une combinaison de ceux-ci ; (ii) phosphate ; (iii) allulose-6-phosphate phosphatase ; (iv) glucose-6-phosphate isomérase ; (v) phosphoglucomutase ou glucose phosphorylase ; et (vi) α-glucanophosphorylase, phosphorylase d'amidon, phosphorylase de maltodextrine, phosphorylase de saccharose, α-amylase, pullulanase, isoamylase, glucoamylase ou sucrase ; ou
(b) un micro-organisme exprimant l'enzyme du point (a) ou une culture du micro-organisme, et

dans laquelle la composition comprend en outre une enzyme phosphatase d'allulose-6-phosphate.

**8.** Procédé pour la production d'allulose, comprenant une étape de conversion de fructose-6-phosphate en allulose-6-phosphate en utilisant la composition pour la production d'allulose telle que décrite dans l'une quelconque des revendications 1 à 4.

**9.** Procédé selon la revendication 8, qui comprend en outre une étape de conversion d'allulose-6-phosphate en allulose par mise en contact de l'allulose-6-phosphate avec une phytase, un micro-organisme exprimant celle-ci ou une culture du micro-organisme.

**10.** Procédé selon la revendication 8, dans lequel l'étape de conversion de fructose-6-phosphate en allulose-6-phosphate est réalisée à une température de réaction de 40 à 70 °C et à un pH de réaction de pH 6 et 8.

**11.** Procédé selon la revendication 8, qui comprend en outre une étape de préparation de fructose-6-phosphate à partir de fructose ou d'une matière contenant du fructose en utilisant de l'hexokinase.

【FIG. 1】

【FIG. 2】

【FIG. 3】

【FIG. 4】

**Temperature property**

【FIG. 5】

【FIG. 6】

【FIG. 7a】

CIFP3E reaction product chromatogram

CDFP3E reaction product chromatogram

【FIG. 7b】

RuFP3E reaction product chromatogram

PkFP3E reaction product chromatogram

**REFERENCES CITED IN THE DESCRIPTION**

**Patent documents cited in the description**

- WO 2018112139 A1